# EUROPÄISCHE PATENTSCHRIFT

(11) **EP 0 837 652 B1**
(45) Veröffentlichungstag und Bekanntmachung des Hinweises auf die Patenterteilung: **10.05.2000**
(21) Anmeldenummer: 96910927.1
(22) Anmeldetag: 25.03.1996
(51) Int. Cl.: A61B 17/06, A61B 17/04

(54) **CHIRURGISCHE NÄHNADEL**
SURGICAL SUTURING NEEDLE
AIGUILLE A COUDRE CHIRURGICALE

(30) Priorität: 10.06.1995 DE 19521228
(43) Veröffentlichungstag der Anmeldung: 29.04.1998
(73) Patentinhaber: Forschungszentrum Karlsruhe GmbH, 76133 Karlsruhe (DE)
(72) Erfinder: WURSTER, Helmut, D-75038 Oberderdingen (DE); TRAPP, Rainer, D-76676 Graben-Neudorf (DE)
(86) Internationale Anmeldenummer: EP9601299
(87) Internationale Veröffentlichungsnummer: WO9641575

(56) Entgegenhaltungen:
- EP-A- 0 649 633
- EP-A- 0 687 446
- WO-A-93/01750
- WO-A-95/01129
- DE-A- 3 136 100
- DE-C- 4 423 881
- US-A- 2 841 150
- US-A- 4 524 771

## Beschreibung

Die Erfindung betrifft eine chirurgische Nähnadel gemäß dem Oberbegriff des Anspruchs 1.

Eine solche Nähnadel ist durch die im Oberbegriff berücksichtigte Entgegenhaltung (PS 41 24 383) bekannt. Die Nähnadel dort hat ebenfalls zwei ausgebildete Spitzen mit denen Gewebe durchstochen werden kann. Sie hat ferner ein Nadelöhr in der Mitte der Nadel, durch das hindurch der Nähfaden gezogen werden kann, um dann am Ende gegen Herausziehen verknotet zu werden. Der Nadelkörper endet an den beiden Enden verjüngt an der jeweiligen Spitze, so daß dort eine Taille besteht, die für das form- und kraftschlüssige Einspannen in dem einen oder andern Maulteil des Nähgeräts nützlich ist. Diese Nadel ist in dem Nähgerät ohne Handberührung von einem zum andern Maulteil übergebbar und wird durch den Haltemechanismus definiert ausgerichtet in dem jeweiligen Maulteil gehalten. Entsprechend der Bewegungsbahn bezüglich dem Nähgerät weist die Nadelachse eine Krümmung (siehe PS 41 24 381) auf, wodurch beim Nähen Gewebeeinrisse vermeidbar werden.

Zwar sitzt die Nadel in dem einen oder andern oder auch gleichzeitig in beiden Maulteilen ausgerichtet, ist aber dennoch nicht gegen Verdrehen in der Halterung hinreichend gesichert, wenn ihr Querschnitt im Bereich der Nadelspitzen kreisrund ist.

Der Erfindung liegt daher die Aufgabe zugrunde, eine chirurgische Nähnadel für insbesondere die minimal invasive Chirurgie bereitzustellen, die zwischen den zwei Maulteilen eines geeigneten Nähgeräts übergeben werden kann, in dem einen oder andern Maulteil definiert ausgerichtet und verdrehsicher gehalten werden kann und mit der zu nähendes Gewebe leicht, bei ausreichender Steifigkeit der Nadel durchdrungen werden kann.

Diese Aufgabe wird durch die im Anspruch 1 gekennzeichneten Merkmale gelöst. Die Nadel hat einen symmetrischen Aufbau. Im hinteren Bereich ihrer beiden kegelförmigen Spitzen setzt eine Ausnehmung an, die senkrecht zur Nadelachse gerade verläuft. Auf diese Ausnehmung kann nun der einfach gestaltete Druckstift im Maulteil gefahren werden, der die im Maulteil anliegende Nadelspitze in die konische Nadelhalterung im Maulteil drückt und fixiert. Dadurch wird die Nadel stets in vorgesehener Lage ausgerichtet gehalten und läßt sich gegen das gerade haltende Maulteil nicht bewegen, selbst beim Durchstechen zäherer Gewebepartien.

Beim häufigen versuchsweise Nähen hat sich herausgestellt, daß ein Kegelwinkel der beiden Nadelspitzen im Bereich von 25 bis 35° optimal ist. Einerseits kann die Nadel hinreichend steif hergestellt werden, andererseits läßt sie dem Chirurgen beim Nähen noch ausreichend Bewegungsraum.

Zweckmäßige Ausgestaltungen sind in den Unteransprüchen 2 bis 5 gekennzeichnet, die helfen, beim Durchziehen der Nadel durch Gewebe die Verletzung auf den Durchstich zu beschränken und Gewebeeinrisse durch z.B. den überstehenden Faden zu vermeiden. Daher sind nach Anspruch 2 sämtliche Kanten der Nadel abgerundet, weist die Nadel im konvexen Bereich des Nadelöhrs eine nutförmige Ausnehmung auf und ist das Nadelöhr im konkaven Bereich zur Aufnahme des Knotens am Faden vertieft. Bei einer taillenförmigen Gestaltung des hinteren Spitzenbereichs an der Nadel kann der Druckstift an seiner Stirn auch gabelförmig gestaltet sein, damit läßt sich ebenfalls ein sehr sicheres Einspannen der Nadel erreichen (Anspruch 4).

Die sohlenartige, gleichartige Ausnehmung jeweils im Bereich im Anschluß an die Nadelspitzen verlaufen in ihrem ebenen Teil in Richtung des mittigen Nadelöhrs unter einem Winkel von 10 bis 30° zur Nadelachse (Anspruch 5). Das hat sich aus Erfahrung als sehr günstig für den Gewebedurchstich erwiesen. Bei einer solchen Neigung zur Nadelachse war die traumatisierende Wirkung am durchstochenen Gewebe am geringsten.

Der Vorteil der Nadel liegt einmal in ihrer einfachen Herstellung und in ihrer konstruktiven Gestaltung der Ausnehmung. Es sind keine spannabhebenden Herstellungsschritte nötig. Die Nadel kann durch Stanz- oder Preßverfahren in hohen Stückzahlen preisgünstig hergestellt werden. Als Halteeinrichtung im Maulteil des Nähgeräts genügt ein kerbenartiges oder ein konisches, der Nadelspitze angepaßtes Widerlager an das die Nadelspitze über den vorgefahrenen Druckstift gedrückt wird. Die Nadel weist im Bereich der Ausnehmung eine dünnste Stelle auf, an der der Druckstift andrückt. Dadurch ist die Nadel auch gegen Herausziehen hinreichend gesichert.

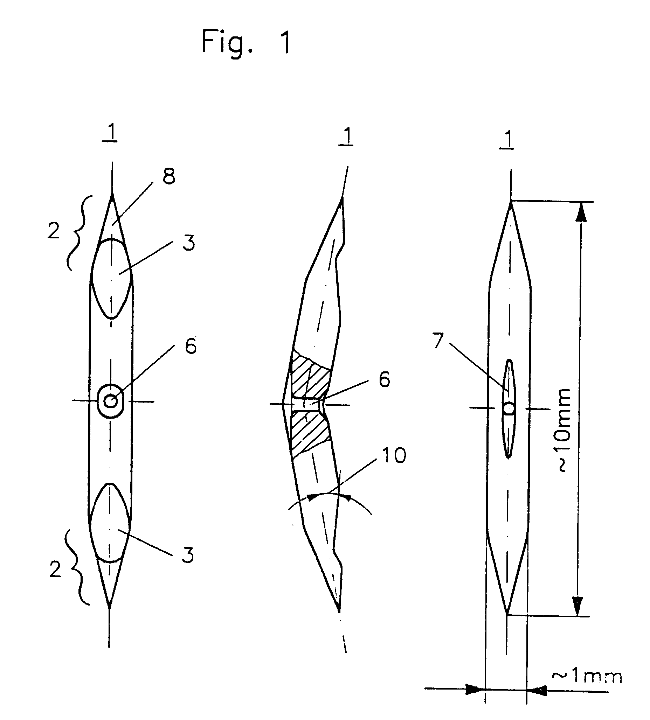

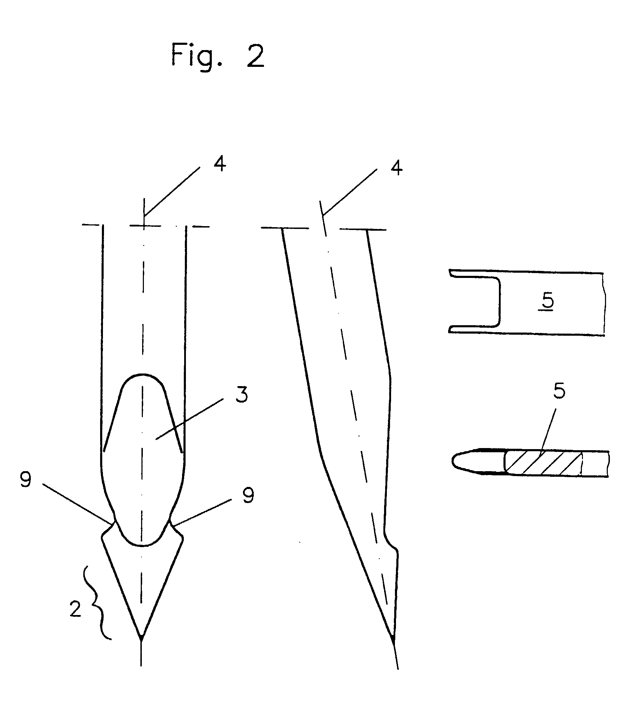

Die Erfindung wird nachstehend an Hand der in der Zeichnung dargestellten Ausführungsform näher erläutert. Es zeigt Figur 1 die Nadel in drei verschiedenen Ansichten und Figur 2 die Gestaltung der Ausnehmung für einen gabelförmigen Druckstift.

Die drei Ansichten der chirurgischen Nähnadel 1 in Figur 1 zeigt die Nadel 1 zunächst in der Draufsicht auf den konkaven Bereich, dann die Seitenansicht und schließlich die Draufsicht auf den konvexen Bereich. Die Nadel 1 ist von symmetrischer Gestalt. Im Bereich der Nadelspitze 2 ist die Ausnehmung 3 angebracht, die eine konkave Form aufweist und unter flachem Winkel 10 von 10° bis 30° in Richtung Nadelmitte hin ausläuft. Diese flache Auslaufen verringert den Einstichwiderstand erheblich gegenüber einer zur Fixierung minimal notwendigen rechteckigen oder halbzylindrischen oder halbkugligen Ausnehmung, bei welcher das Gewebe beim Durchstechen in der Ausnehmung hängen bleibt und anreißt und dadurch einen höheren Durchstechwiderstand bildet. Auf diese Fläche der Ausnehmung 3 drückt der Druckstift 5 des Nähgerätemaulteils, in dem die Nadel gerade eingespannt ist, und zwar zentriert auf die dünnste Stelle der Ausnehmung, so daß damit ein axiales Verrücken verhindert wird. Gegen seitliches Verrücken genügt, wenn die Nadel 1 mit ihrem Spitzenbereich in eine kerpenartige Vertiefung im Maulteil des Nähgeräts aufliegt. Die Nadelspitze muß also nicht formschlüssig gehalten werden. Das bedeutet einen erheblich geringeren konstruktiven Aufwand auf der Maulteilseite.

Die Seitenansicht in Figur 1 zeigt den Bereich der Nadelöhre 6 geschnitten. Im Nadelrücken, der konvexen Seite, befindet sich dort eine längliche Ausnehmung 7, in die sich der eingefädelte Faden beim Gewebedurchstich einlegt. Der Knoten am Fadenende gegen Herausziehen des Fadens legt sich auf der Bauchseite oder konkaven Seite der Nadel 1 in die Einsenkung der Nadelöhre 6 an. Somit sind gewebeverletzende Konturen bis auf die beiden Nadelspitzen 2 unterbunden. In einfacher Form hat die Nadel 1 auf der Rückenseite im Bereich der Öhre 6 lediglich eine Abflachung statt der länglichen Vertiefung.

Die Darstellung der beiden Ausnehmungen 3 und der länglichen Ausnehmung 7 sollen hier ortshinweisend verstanden werden. Es sind nämlich alle Kanten der Nadel 1 abgerundet, um abgesehen vom Gewebeein- und -durchstich nicht weiter verletzend zu wirken.

Zur Verdeutlichung der Größenverhältnisse sind im rechten, dritten Bild der Figur 1 die ungefähren Außenmasse der Nadel angegeben. Die Nadelspitzen haben bei dieser Ausführungsform einen Kegelwinkel 8 von etwa 30°.

Die Darstellung in Figur 2 zeigt eine andere zuverlässige Art und Weise, wie die Nähnadel 1 mit einer ihrer beiden Spitzen 2 in eines der beiden Maulteile des verwendeten Nähgeräts eingespannt werden kann. Im Bereich der Ausnehmung 3 hat die Nadel 1 hierzu eine taillenförmige Einschnürung 9 symmetrisch zur Nadelachse 4, dort greift der Druckstift 5 mit seinem gabelförmigen Ende ein. Er ist auf dieser Höhe im Schnitt angedeutet. Das Bild darüber zeigt den Druckstift 5 voll in der Draufsicht. Auch hier wird der Spitzenbereich über den Druckstift 5 gegen die kerbenförmige oder konische Auflage im Maulteil gedrückt, so daß ein seitliches Verschieben oder Verdrehen ausgeschlossen ist. Das Umfassen der Taille hinter der Nadelspitze 2 durch den gabelförmigen Druckstift 5 verhindert das axiale Verrücken der eingespannten Nadel 1. Auch an dieser Ausführungsform der Nadel 1 sind sämtliche Kanten abgerundet, um die Verletzung des Gewebes am Durchstichort auf ein Minimum zu beschränken.

### Bezugszeichenliste

- 1: Nähnadel, Nadel
- 2: Nadelspitze, Spitze
- 3: Ausnehmung
- 4: Nadelachse, Achse
- 5: Druckstift
- 6: Nadelöhr, Öhr
- 7: Nut, Ausnehmung
- 8: Winkel
- 9: Einschnürung
- 10: Winkel

## Patentansprüche

1. Chirurgische Nähnadel (1), deren beide Enden als Nadelspitze (2) ausgebildet sind, die in ihrer Mitte ein Nadelöhr (6) zur Durchführung und Befestigung eines chirurgischen Nähfadens hat, die mit ihren Spitzen (2) in die beiden Maulteile eines chirurgischen Nähgeräts einspannbar oder von einem zum ändern Maulteil ohne Handberührung übergebbar ist und die eine Krümmung entsprechend oder angenähert der Bewegungsbahn beim Nähen aufweist,
dadurch gekennzeichnet, daß
die Nadel (1) in ihren beiden Spitzenbereichen je eine gleichartige Ausnehmung (3) aufweist, die eine konkave Form hat und unter einem flachen Winkel von 10 bis 30° in Richtung Nadelmitte hin ausläuft, auf die ein Druckstift (5) im Maulteil derart mit seinem Ende drücken kann, daß die Nadel (1) form- und kraftschlüssig, in definierter Ausrichtung in dem jeweiligen Maulteil oder gleichzeitig in beiden gehalten werden kann,
die beiden Spitzen (2) der Nadel (2) einen Kegelwinkel (8) zwischen 25 und 35° aufweist, wodurch ein weitestgehend atraumatisches Durchstechen von Gewebe gegeben ist.

2. Chirurgische Nähnadel nach Anspruch 1,
dadurch gekennzeichnet, daß
sämtliche Kanten der Nadel (1) abgerundet sind.

3. Chirurgische Nähnadel nach Anspruch 2,
dadurch gekennzeichnet, daß
die Nadel (1) im konvexen Bereich des Nadelöhrs (6) eine Nut (7) enthält, in welcher der Faden beim Durchziehen zu liegen kommt, und im konkaven Bereich des Nadelöhrs (6) eingesenkt ist.

4. Chirurgische Nähnadel nach Anspruch 3,
dadurch gekennzeichnet, daß
die Berandung der beiden Ausnehmungen (3) taillenförmig und symmetrisch zur Nadelachse (4) verlaufen, so das ein gabelförmig ausgebildeter Druckstift (5) in der Taille die Nadelspitze (2) aufnehmen und fixieren kann.

5. Chirurgische Nähnadel nach Anspruch 4,
dadurch gekennzeichnet, daß
die Sohle der Ausnehmung (3) zur Nadelfixierung in Richtung Nadelmitte unter einem flachen Winkel (10) von 10 bis 30° zur Nadelachse verläuft, wodurch der Durchstechwiderstand klein gehalten wird.

## Claims

1. Surgical suturing needle (1), the two ends of which are configured as needle point (2), which has in its centre a needle eye (6) for passing therethrough and securing a surgical suturing thread, said needle being clampable with its points (2) in the two mouthpieces of a surgical suturing apparatus or being transferable from one mouthpiece to the other without manual contact, and said needle has a curvature corresponding to, or substantially identical to, the path of movement during the suturing operation, characterised in that the needle (1) has, in each of its two point regions, an identical recess (3) which has a concave configuration and extends towards the needle centre at a small angle of between 10° and 30°, a pressure pin (5) in the mouthpiece being able to press with its end upon said recess in such a manner that the needle (1) can be retained in a form- and force-locking manner, in a specific alignment in the respective mouthpiece or simultaneously in both mouthpieces, and in that the two points (2) of the needle (1) have a taper angle (8) of between 25° and 35°, whereby a largely atraumatic piercing of tissue is effected.

2. Surgical suturing needle according to claim 1, characterised in that all of the edges of the needle (1) are rounded.

3. Surgical suturing needle according to claim 2, characterised in that the needle (1) includes, in the convex region of the needle eye (6), a groove (7) in which the thread comes to lie when being drawn therethrough, and said needle is depressed in the concave region of the needle eye (6).

4. Surgical suturing needle according to claim 3, characterised in that the boundary of the two recesses (3) extends in a waistlike manner and symmetrically relative to the needle axis (4), so that a bifurcated pressure pin (5) can accommodate and secure the needle point (2) in the waist.

5. Surgical suturing needle according to claim 4, characterised in that, to secure the needle, the base of the recess (3) extends in the direction of the needle centre at a small angle (10) of between 10° and 30° relative to the needle axis, whereby the resistance to piercing is kept low.

## Revendications

1. Aiguille à coudre (1) chirurgicale, dont les deux extrémités sont réalisées sous la forme de pointe d'aiguille (2) et qui comporte en son centre un chas d'aiguille (6) pour permettre le passage et la fixation d'un fil de couture chirurgical, qui peut être enserrée par ses pointes (2) dans les deux parties de bec d'un appareil de couture chirurgical, ou bien peut être passée d'une partie de bec à l'autre, sans contact manuel, et présentant une courbure correspondant ou s'approchant de la trajectoire de déplacement lors de la couture,
caractérisée en ce que
- l'aiguille (1) présente, dans chacune de ses deux zones de pointe, un évidement (3) de même genre, ayant une forme concave et allant en évoluant sous un angle plat de 10 à 30° en direction du centre de l'aiguille, évidement sur lequel une tige de pressage (5) peut être pressée par son extrémité dans la partie de bec, de manière que l'aiguille (1) puisse être maintenue avec une liaison par ajustement de forme et avec interaction de forces, selon une orientation définie, dans la partie de bec respective, ou simultanément dans les deux ;
- les deux pointes (2) de l'aiguille (1) présentant un angle de conicité (8) compris entre 25 et 35°, faisant qu'on peut effectuer un transpercement largement traumatique des tissus.

2. Aiguille à coudre chirurgicale selon la revendication 1,
caractérisée en ce que
l'ensemble des arêtes de l'aiguille (1) sont arrondies.

3. Aiguille à coudre chirurgicale selon la revendication 2,
caractérisée en ce que
l'aiguille (1) comporte, dans la zone convexe du chas d'aiguille (6), une rainure (7) dans laquelle le fil vient se presser lors de l'enfilage et est noyé dans la zone concave du chas d'aiguille (6).

4. Aiguille à coudre chirurgicale selon la revendication 3,
caractérisée en ce que
la bordure des deux évidements (3) est en forme d'entaille et s'étend symétriquement par rapport à l'axe (4) de l'aiguille, si bien qu'une tige de pressage (5), réalisée en forme de fourche, peut être logée et fixée dans l'entaille.

5. Aiguille à coudre chirurgicale selon la revendication 4,
caractérisée en ce que
la sole de l'évidement (3) pour la fixation de l'aiguille s'étend en direction du centre de l'aiguille sous un angle plat (10) de 10 à 30° par rapport à l'axe d'aiguille, faisant que la résistance au transpercement est maintenue à une faible valeur.
